# EUROPÄISCHE PATENTSCHRIFT

(11) **EP 2 405 862 B1**
(45) Veröffentlichungstag und Bekanntmachung des Hinweises auf die Patenterteilung: **07.11.2012**
(21) Anmeldenummer: 11707078.9
(22) Anmeldetag: 16.02.2011
(51) Int. Cl.: A61F 2/18

(54) **TESTEINRICHTUNG MIT THERMO-DUMMY FÜR GEHÖRKNÖCHELCHENPROTHESE MIT FORMGEDÄCHTNIS**
TEST APPLIANCE WITH THERMAL DUMMY FOR AN AUDITORY OSSICLE PROSTHESIS WITH SHAPE MEMORY
DISPOSITIF D'ESSAI COMPORTANT UN OBJET D'IMITATION THERMIQUE POUR PROTHÈSE DES OSSELETS DE L'OUÏE À MÉMOIRE DE FORME

(30) Priorität: 12.05.2010 DE 102010020412
(43) Veröffentlichungstag der Anmeldung: 18.01.2012
(73) Patentinhaber: Heinz Kurz GmbH Medizintechnik, 72144 Dusslingen (DE)
(72) Erfinder: STEINHARDT, Uwe, 72145 Hirrlingen (DE)
(74) Vertreter: Kohler Schmid Möbus
(86) Internationale Anmeldenummer: PCT/EP2011/000727
(87) Internationale Veröffentlichungsnummer: WO 2011/141072

(56) Entgegenhaltungen:
- WO-A1-02/069850
- DE-B3-102007 062 151
- US-A- 5 615 770

## Beschreibung

Die Erfindung betrifft eine Testeinrichtung mit einer Gehörknöchelchenprothese zur Implantation ins Mittelohr, die mindestens ein Glied oder Teile eines Gliedes der menschlichen Gehörknöchelchenkette ersetzt oder überbrückt, wobei die Gehörknöchelchenprothese an ihrem einen Ende ein erstes Befestigungselement zur mechanischen Verbindung mit dem Trommelfell oder einem Glied der Gehörknöchelchenkette, insbesondere mit dem Ambossfortsatz oder mit dem Hammergriff, und an ihrem anderen Ende ein zweites Befestigungselement zur mechanischen Verbindung mit einem weiteren Glied oder Teilen eines Gliedes der Gehörknöchelchenkette oder direkt mit dem Innenohr sowie ein die beiden Befestigungselemente Schall leitend miteinander verbindendes Verbindungselement aufweist, und wobei zumindest Teile der Gehörknöchelchenprothese aus einem Material mit Formgedächtnis (= memory effect) hergestellt sind, welche bei der Implantation der Gehörknöchelchenprothese im Mittelohr einer Form verändernden thermischen Behandlung unterzogen werden.

Derartige Gehörknöchelchenprothesen, bei denen mindestens ein Befestigungselement aus Nitinol besteht und intraoperativ im Mittelohr durch thermische Behandlung in seine endgültige Form gebracht wird, sind beispielsweise bekannt aus der WO 02/069850 A1 oder aus der US 6,554,861 B2.

Gehörknöchelchenprothesen werden verwendet, um bei ganz oder teilweise fehlenden oder geschädigten Gehörknöchelchen des menschlichen Mittelohrs den Schall bzw. das Schallsignal vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird.

Drei besonders häufig verwendete Arten von Gehörknöchelchenprothesen sind die Steigbügel-Prothesen, die Partial-Prothesen und die Total-Prothesen. Steigbügel-Prothesen (= Stapes-Prothesen) werden am Amboss fixiert und ragen über einen Stempel (= Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte.

Die gattungsgemäßen Gehörknöchelchenprothesen haben darüber hinaus gemeinsam, dass ihre Befestigung an einem Glied der Gehörknöchelchenkette während der Implantation dadurch bewirkt oder zumindest unterstützt wird, dass die gesamte Prothese oder wenigstens das entsprechende Befestigungselement aus einem Material mit Formgedächtnis hergestellt wurde, welches präoperativ einer Verformung unter definierten Temperaturbedingungen unterzogen wurde. Um nun ein beispielsweise als Schlinge ausgelegtes Befestigungselement dauerhaft zu befestigen, wird die Schlinge zunächst relativ grob um den winzigen Teilbereich des entsprechenden Gehörknöchelchens herum gelegt, genau positioniert und anschließend thermisch - meist mit einem Laser oder einer elektrischen Einrichtung - derart behandelt, dass durch gezielte Verformung des Memory-Materials ein endgültiger und - bei Körpertemperatur dann auch permanenter - Verschluss der Schlinge um das Knöchelchen herum erfolgt. Analog wird auch bei anders geformten Befestigungselementen vorgegangen.

Problematisch ist dabei jedoch, dass beim Einbringen von zu hoher Lichtleistung bzw. elektrischer Energie ungewollt eine zu große lokale Aufheizung und damit eine zu hohe Temperatur im Bereich des Befestigungselements erzeugt wird, die sich auf das Gehörknöchelchen überträgt und schnell zu einer Schädigung oder gar Nekrotisierung des empfindlichen Körpergewebes am entsprechenden Abschnitt des Gehörknöchelchens führen kann. Die ganze Operation wäre dann im Endergebnis möglicherweise sogar kontraproduktiv.

Natürlich machen die Hersteller von gattungsgemäßen Mittelohrprothesen in der Regel Angaben über optimale Verfahrensparameter bei der "Verarbeitung" ihrer Prothesen mit memory effect. Aber allein schon wegen der stark differierenden Geräte der Anwender zur thermischen Behandlung vor Ort können diese Angaben lediglich sehr breite und damit oftmals nicht sonderlich aussagekräftige Bereiche umfassen. Außerdem hilft es einem Operateur in der Praxis herzlich wenig, wenn ihm gesagt wird, welche lokale Maximaltemperatur er bei der thermischen Behandlung nicht überschreiten darf. Er weiß ohnehin, dass Eiweiß ab etwa 60°C koaguliert und Körpergewebe dauerhaft geschädigt werden kann. Vielmehr muss er eine geeignete Einstellung der Leistungsabgabe seines individuell vorhandenen Gerätes finden und außerdem auch die für seine Zwecke optimale Stelle der Energiezufuhr auf die Gehörknöchelchenprothese herausfinden, bevor er die Operation im Mittelohr durchführt.

Aufgabe der vorliegenden Erfindung ist es demgegenüber, mit möglichst einfachen technischen Mitteln unaufwändig und kostengünstig eine Testeinrichtung mit Mittelohrprothese der eingangs beschriebenen Art bereit zu stellen, die dem Operateur eine unkomplizierte Möglichkeit eröffnet, intraoperativ kurz vor der eigentlichen Implantation der Mittelohrprothese eine für seine vorhandenen thermischen Behandlungsgeräte optimale Parametereinstellung zu ermitteln, ohne dass die Mittelohrprothese selbst auch nur berührt werden muss oder ungewünscht irgendwie verändert werden könnte.

Erfindungsgemäß wird diese Aufgabe auf ebenso überraschend einfache wie wirkungsvolle Weise dadurch gelöst, dass die Testeinrichtung eine Vorrichtung zur Ermittlung einer optimierten Leistungseinstellung und/oder Energieapplikationsstelle für die Bearbeitung der intraoperativ thermisch zu behandelnden Teile der Gehörknöchelchenprothese umfasst, und dass diese Vorrichtung eine der Gehörknöchelchenprothese nachgebildete Prothesenattrappe enthält, welche zumindest in denjenigen Bereichen, in denen die zugehörige Gehörknöchelchenprothese intraoperativ thermisch behandelt werden soll, mit der Gehörknöchelchenprothese hinsichtlich Material, geometrischer Gestaltung und Herstellungsverfahren identisch aufgebaut ist.

Auf diese Weise kann der Operateur zunächst die optimalen Parameter der für die Gehörknöchelchenprothese hinterher in situ vorgesehenen thermischen Behandlung im Mittelohr bereits vorher an der Prothesenattrappe mit seinen vorhandenen Geräten ermitteln und geplante Handlungsabläufe statt im Mittelohr problemlos außerhalb austesten, ohne dabei die originale Prothese auch nur berühren zu müssen und dabei eventuell ungewollt zu verformen oder gar zu beschädigen. Die so ermittelten Parameter entsprechen sehr genau den für die Original-Prothese gültigen Parametern, weil die Prothesenattrappe erfindungsgemäß zumindest an den Stellen der geplanten thermischen Behandlung mit der Original-Prothese identisch übereinstimmt.

Ein Hauptproblem, das bei jeder Rekonstruktion der menschlichen Gehörknöchelchenkette auftaucht, ist die Auswahl der richtigen Prothesenlänge. Anatomisch bedingt, variieren die jeweils erforderlichen Längen in einem Spektrum von mehreren Millimetern. Daher muss beim operativen Einsetzen einer Gehörknöchelchenprothese entweder eine ausreichend große Auswahl von Prothesen unterschiedlicher axialer Länge bereitgehalten werden oder die verwendete Gehörknöchelchenprothese muss während der Operation ausgehend von einer maximalen Ausgangslänge auf die erforderliche axiale Endlänge gebracht werden können. Derartige Längen variablen Gehörknöchelchenprothesen sind in vielen unterschiedlichen Ausgestaltungen im Stand der Technik bekannt.

Bei einer bevorzugten Klasse von Ausführungsformen der erfindungsgemäßen Testeinrichtung weist daher die Gehörknöchelchenprothese eine solche Einrichtung zur Variation ihrer Länge auf, die zumeist im Bereich des Verbindungselements zwischen den beiden Enden der Prothese angeordnet ist. Die Gehörknöchelchenprothese und ihre zugehörige Prothesenattrappe sind dann zumindest im Bereich der Einrichtung zur Längenvariation aus einem Material mit Formgedächtnis hergestellt.

Alternativ oder zusätzlich können bei weiteren Ausführungsformen der Erfindung die Gehörknöchelchenprothese sowie ihre zugehörige Prothesenattrappe zumindest im Bereich des jeweiligen ersten Befestigungselements aus einem Material mit Formgedächtnis hergestellt sein, um prä-operativ die optimalen Parameter für das oben beschriebene intraoperative Schließen des Befestigungselements ermitteln zu können.

Bei einer besonders einfachen Klasse von Ausführungsformen der erfindungsgemäßen Testeinrichtung sind die Gehörknöchelchenprothese sowie ihre zugehörige Prothesenattrappe hinsichtlich Material und geometrischer Gestaltung vollständig identisch aufgebaut. Es müssen dem Operateur also lediglich zwei gleiche Mittelohrprothesen vorliegen, von denen er eine dann als Prothesenattrappe verwenden kann, während er die andere anschließend im Mittelohr implantiert.

Eine weitere erhebliche Verbesserung der Handhabung wird bei Ausführungsformen erreicht, bei welchen die Testeinrichtung eine Einrastvorrichtung enthält, mit welcher die Prothesenattrappe zumindest in einer Bearbeitungsposition fixierbar ist. Die starre und unverrückbare Lage der Prothesenattrappe während der Ermittlung von optimalen Parametern erleichtert nicht nur die Arbeit, sondern erhöht auch die Qualität der aufgefundenen Parameter.

Besonders bevorzugte und für den Benutzer komfortable Ausführungsformen der erfindungsgemäßen Testeinrichtung zeichnen sich dadurch aus, dass eine Halterungsvorrichtung vorgesehen ist, welche die Prothesenattrappe verschwenkbar aufnehmen kann. Damit kann nicht nur eine stabile und gut zugängliche Positionierung der Prothesenattrappe während der Ermittlung der optimalen Behandlungsparameter, sondern auch ein schonender und beschädigungsfreier Transport zum "Einsatzort" sichergestellt werden.

Besonders bequem handhabbar und einfach in der Herstellung sind Weiterbildungen der erfindungsgemäßen Testeinrichtung, bei denen die Halterungsvorrichtung als Platte aus dünnem Blech gefertigt ist und eine mit der Platte verwindbar verbundene Einhängevorrichtung zum Einhängen eines Endes der Prothesenattrappe umfasst.

Vorteilhaft im Hinblick auf einen beschädigungsfreien Transport sind auch Weiterbildungen der oben beschriebenen Ausführungsformen, die sich dadurch auszeichnen, dass die Halterungsvorrichtung eine Einrichtung zur Transportsicherung aufweist, welche seitlich von der Halterungsvorrichtung abstehende grätenförmige Stege umfasst.

Die Handhabung der erfindungsgemäßen Testeinrichtung kann Weiterbildungen der obigen Ausführungsformen noch weiter erleichtert werden, wenn die Halterungsvorrichtung derart gestaltet ist, dass sie auch die zur Prothesenattrappe gehörende Gehörknöchelchenprothese aufnehmen kann.

Besonders bevorzugt sind auch Weiterbildungen, bei denen die Halterungsvorrichtung optisch kodiert und/oder beschriftet ist, wobei die Kodierung oder Beschriftung technische Informationen bezüglich der Gehörknöchelchenprothese und/oder der zugehörigen Prothesenattrappe und/oder Herstellerangaben enthält, so dass diese wichtigen Daten nicht erst in einem Handbuch oder Begleitzettel zur Testeinrichtung nachgeschlagen werden müssen, sondern unmittelbar bei der Handhabung der Testeinrichtung zur Verfügung stehen.

Ebenso vorteilhaft und daher bevorzugt sind Ausführungsformen der erfindungsgemäßen Testeinrichtung, die sich dadurch auszeichnen, dass die Gehörknöchelchenprothese und/oder die zugehörige Prothesenattrappe optisch kodiert und/oder beschriftet ist, und dass die Kodierung oder Beschriftung technische Informationen über die Gehörknöchelchenprothese und/oder der Prothesenattrappe enthält.

In beiden Fällen ist es fertigungstechnisch am einfachsten, wenn die Kodierung und/oder Beschriftung mittels Laserbehandlung und/oder Anodisierung erzeugt wird.

Von besonderem praktischen Vorteil sind Weiterbildungen, bei denen die technischen Informationen der Kodierung und/oder Beschriftung einen empfohlenen Anfangswert für die elektrische Leistung oder Lichtleistung enthalten, welcher zu Beginn der Ermittlung von optimierten Bearbeitungsparametern der intraoperativ thermisch zu behandelnden Teile der Gehörknöchelchenprothese mit Hilfe einer Testbehandlung der Prothesenattrappe angewendet werden soll.

In der Praxis bewähren sich auch Ausführungsformen der erfindungs-gemäßen Testeinrichtung, bei denen zumindest Teile der Gehörknöchelchenprothese und der zugehörigen Prothesenattrappe aus einer Nickel-Titan-Legierung, insbesondere aus Nitinol hergestellt sind.

Die Gehörknöchelchenprothese der erfindungsgemäßen Testeinrichtung kann als passive Prothese ausgestaltet sein. Alternativ kann aber bei Ausführungsformen der Erfindung die Gehörknöchelchenprothese auch mit einem aktiven Vibrationsteil eines aktiven, teilweise implantierbaren Hörgeräts verbunden werden. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Gehörknöchelchenprothese mit der Außenwelt mittels einer weiter unten beschriebenen Beschichtung keine Probleme durch einen erhöhten Bakterieneintrag in den Bereich des Mittelohres verursacht, wenn die Beschichtung entsprechend antibakteriell ausgestaltet ist.

Je nach dem individuellen Defekt, der bei einem Patienten durch den Einsatz der in der erfindungsgemäßen Testeinrichtung enthaltenen Gehörknöchelchenprothese behoben oder zumindest in seinen Auswirkungen gelindert werden soll, wird der Aufbau der Prothese entsprechend gestaltet sein. In einigen Ausführungsformen der Erfindung wird das erste Befestigungselement eine zur Anlage am Trommelfell ausgebildete Kopfplatte umfassen. Bei vielen Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine Klasse von Ausführungsformen der mit der Testeinrichtung verwendeten Gehörknöchelchenprothese zeichnet sich dadurch aus, dass das zweite Befestigungselement als Platte, als Hülse, als Schlinge, als geschlossene Glocke, als einfach oder mehrfach geschlitzte Glocke oder als Clip zur mechanischen Verbindung mit einem weiteren Glied der Gehörknöchelchenkette ausgebildet ist.

Bei Weiterbildungen dieser Ausführungsformen ist die Prothese über eine Kopfplatte einerseits am Trommelfell und über das zweite Befestigungselement andererseits am Amboss oder am Steigbügel befestigt.

Alternative Ausgestaltungen können vorsehen, dass die Gehörknöchelchenprothese an ihrem das zweite Befestigungselement tragenden Ende mittels Perforation der Steigbügelfußplatte (= Stapedektomie bzw. Stapedotomie) und/oder mittels Eröffnung der menschlichen Hörschnecke (= Cochleotomie) direkt an das Innenohr angekoppelt ist, insbesondere über einen Kolben.

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen einer Kopfplatte und dem Verbindungselement kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse postoperative Mobilität aufweist, so dass sich die Kopfplatte postoperativ selbstständig der Position des Trommelfells angleichen kann. Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen.

In der Regel wird bei der Gehörknöchelchenprothese das Verbindungselement zwischen den beiden Befestigungselementen als länglicher Schaft gestaltet sein, wie dies an sich aus dem Stand der Technik wohlbekannt ist. Um die oben erörterte erhöhte Flexibilität bzw. Variabilität der Prothese zu erreichen - wie beispielsweise ausführlich in der EP 1 181 907 B1 beschrieben - kann bei einer besonders bevorzugten Ausführungsform der Erfindung im länglichen Verbindungselement mindestens ein Kugelgelenk vorgesehen sein. Vorteilhaft im Hinblick auf eine besonders hohe postoperative Beweglichkeit der Prothese sind Weiterbildungen, bei denen der längliche Schaft eine Vielzahl von aneinander angrenzenden weiteren Drehelementen, vorzugsweise eine Kugelgelenkkette, umfasst.

Teile der Gehörknöchelchenprothese sowie der zugehörigen Prothesenattrappe in der erfindungsgemäßen Testeinrichtung können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon, insbesondere eines der Befestigungselemente, aus einem Material mit superelastischen Eigenschaften, vorzugsweise wiederum aus Nitinol hergestellt sind, was per se beispielsweise aus der eingangs zitierten WO 02/069850 A1 oder aus der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Ein als Kopfplatte ausgebildetes Befestigungselement sollte bei der in der erfindungsgemäßen Testeinrichtung enthaltenen Gehörknöchelchenprothese grundsätzlich eine Wachstums fördernde Beschichtung, ein direkt ins Innenohr führendes, etwa in Form eines Kolbens ausgebildetes Befestigungselement hingegen eine Wachstums hemmende Beschichtung aufweisen.

Besonders bevorzugt ist eine Ausführungsform, bei der die Massenverteilung der einzelnen Teile der Prothese in Abhängigkeit von einem gewünschten, vorgegebenen oder vorgebbaren Frequenzgang der Schallleitung im Mittelohr berechnet ist. Damit lässt sich ohne großen zusätzlichen technischen Aufwand gewissermaßen ein mechanisches Tuning der Schallfortpflanzungseigenschaften mittels einer individuellen ausgestalteten Gehörknöchelchenprothese erreichen.

Ein solcher Tuning-Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Im Einzelnen zeigen:
- Fig. 1: eine Ausführungsform der erfindungsgemäßen Testeinrichtung mit einer neben einer zugehörigen Gehörknöchelchenprothese auf einer Halterungsvorrichtung im Bereich einer Einrastvorrichtung verschwenkbar aufgenommenen Prothesenattrappe in einer schematischen räumlichen Darstellung von schräg oben;
- Fig. 2a: eine Detailansicht der Ausführungsform von Fig. 1 im Bereich der Einrastvorrichtung;
- Fig. 2b: eine Detailansicht der Ausführungsform von Fig. 1 mit der gesamten Prothesenattrappe in einer schematischen räumlichen Darstellung von schräg oben um etwa 180° gegenüber Fig. 1 verdreht;
- Fig. 3a-3e: die Ausführungsform von Fig. 1 in schematischer Draufsicht (a) von oben, (b) von unten, (c) von einer Stirnseite I, (d) von der Gegen-Stirnseite II und (e) von einer Längsseite III;
- Fig. 4a: die Ausführungsform von Fig. 1 in schematischer räumlicher Darstellung von schräg oben mit entnommener Gehörknöchelchenprothese und aufgerichteter und mit der Halterungsvorrichtung verrasteter Prothesenattrappe;
- Fig. 4b: wie Fig. 4a, aber um etwa 180° verdreht aus der Perspektive der gegenüber liegenden Längsseite;
- Fig. 5a: die Halterungsvorrichtung der Ausführungsform von Fig. 1, jedoch ohne Gehörknöchelchenprothese und ohne Prothesenattrappe sowie mit der Einrastvorrichtung und einer verschwenkbaren Halterung für die Gehörknöchelchenprothese jeweils im unverschwenkten Zustand;
- Fig. 5b: die Halterungsvorrichtung von Fig. 5a in einer schematischen Ansicht von unten;
- Fig. 5c: die Halterungsvorrichtung von Fig. 5a mit der verschwenkbaren Halterung für die Gehörknöchelchenprothese im verschwenkten Zustand; und
- Fig. 5d: die Halterungsvorrichtung von Fig. 5c in einer schematischen Ansicht von unten.

Die in den Figuren der Zeichnung schematisch dargestellte Ausführungsform der erfindungsgemäßen **Testeinrichtung 1** mit einer **Gehörknöchelchenprothese 2** zur Implantation ins Mittelohr, die an ihrem einen Ende ein **erstes Befestigungselement 2a** zur mechanischen Verbindung mit dem Trommelfell oder einem Glied der Gehörknöchelchenkette und an ihrem anderen Ende ein **zweites Befestigungselement 2b** zur mechanischen Verbindung mit einem weiteren Glied der Gehörknöchelchenkette oder direkt mit dem Innenohr sowie ein die beiden Befestigungselemente 2a, 2b Schall leitend miteinander verbindendes **Verbindungselement 2c** aufweist, und wobei zumindest Teile der Gehörknöchelchenprothese 2 aus einem Material mit Formgedächtnis (= memory effect) hergestellt sind, welche bei der Implantation der Gehörknöchelchenprothese 2 im Mittelohr einer Form verändernden thermischen Behandlung unterzogen werden, zeichnet sich erfindungsgemäß dadurch aus, dass die Testeinrichtung 1 eine Vorrichtung zur Ermittlung einer optimierten Leistungseinstellung und/oder Energieapplikations-Stelle für die Bearbeitung der intraoperativ thermisch zu behandelnden Teile der Gehörknöchelchenprothese 2 umfasst, und dass die Vorrichtung eine der Prothese 2 nachgebildete **Prothesenattrappe 3** enthält, welche zumindest in denjenigen Bereichen, in denen die zugehörige Prothese 2 intraoperativ thermisch behandelt werden soll, mit der Gehörknöchelchenprothese 2 hinsichtlich Material, geometrischer Gestaltung und Herstellungsverfahren identisch aufgebaut ist.

Die Gehörknöchelchenprothese 2 der erfindungsgemäßen Testeinrichtung 1 sowie ihre zugehörige Prothesenattrappe 3 sind bei der in Zeichnung gezeigten Ausführungsform der Erfindung zumindest im Bereich des ersten Befestigungselements 2a aus einem Material mit Formgedächtnis hergestellt. Bei in der Zeichnung nicht dargestellten Ausführungsformen der Erfindung kann die Prothese aber auch eine Einrichtung zur Variation ihrer Länge aufweisen, wobei dann die Prothese sowie ihre zugehörige Prothesenattrappe zumindest im Bereich der Einrichtung zur Längenvariation aus einem Material mit Formgedächtnis hergestellt sind. Bei ebenfalls nicht in der Zeichnung dargestellten Ausführungsformen der erfindungsgemäßen Testeinrichtung können die Gehörknöchelchenprothese sowie ihre zugehörige Prothesenattrappe hinsichtlich Material und geometrischer Gestaltung vollständig identisch aufgebaut sein.

Als bevorzugtes Material mit Formgedächtnis (= memory effect) kommt bei vielen Ausführungsformen der Erfindung eine Nickel-Titan-Legierung, insbesondere Nitinol zum Einsatz.

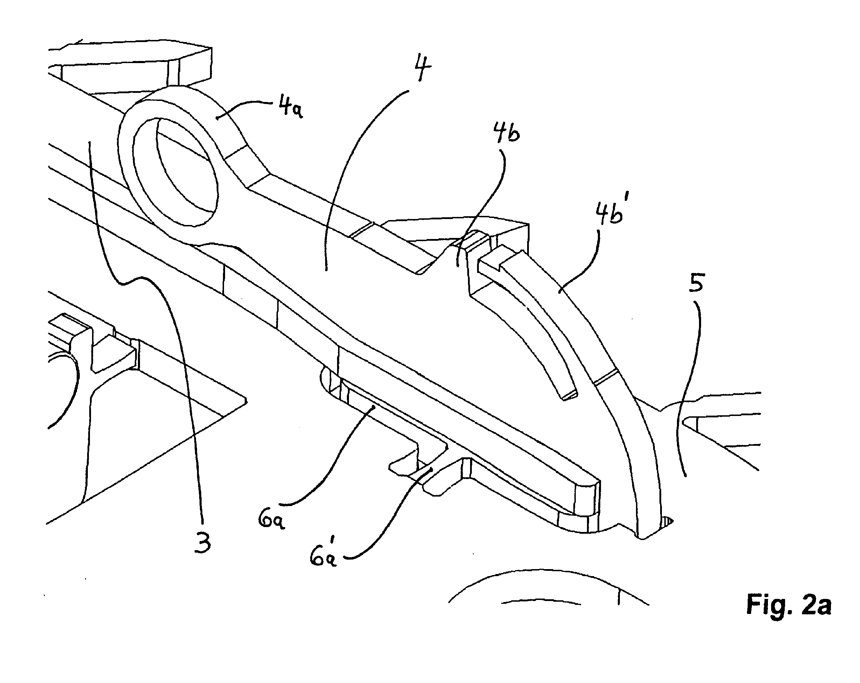

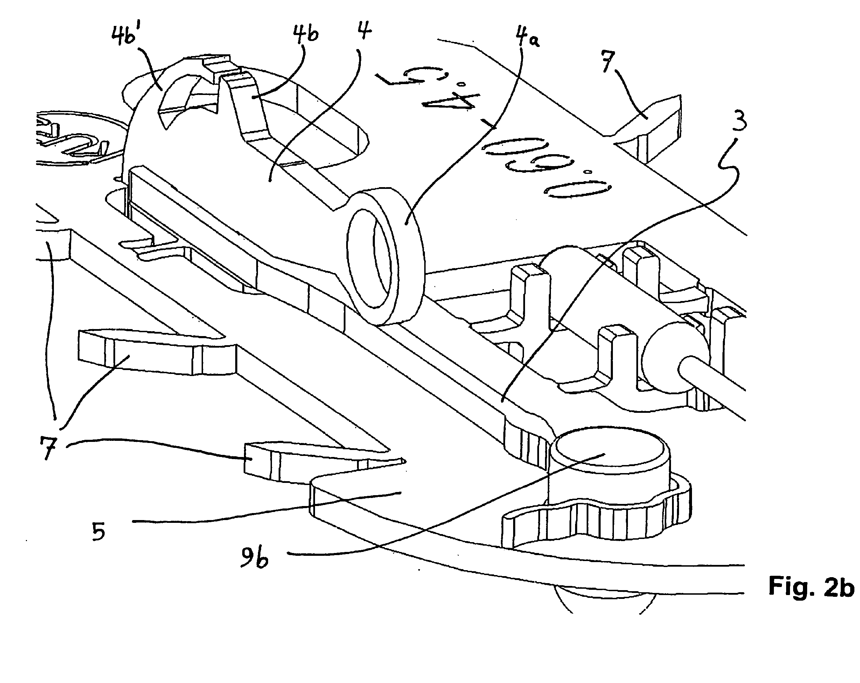

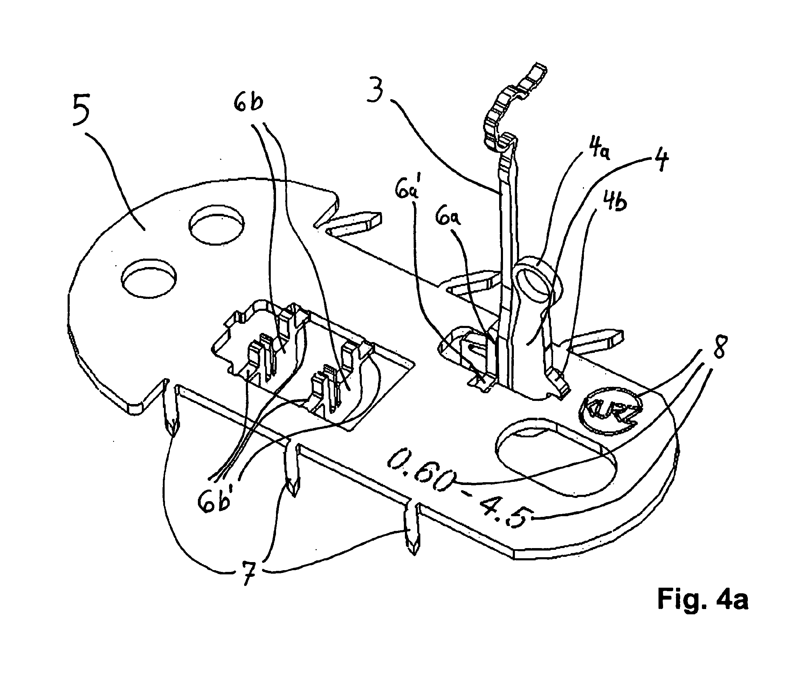

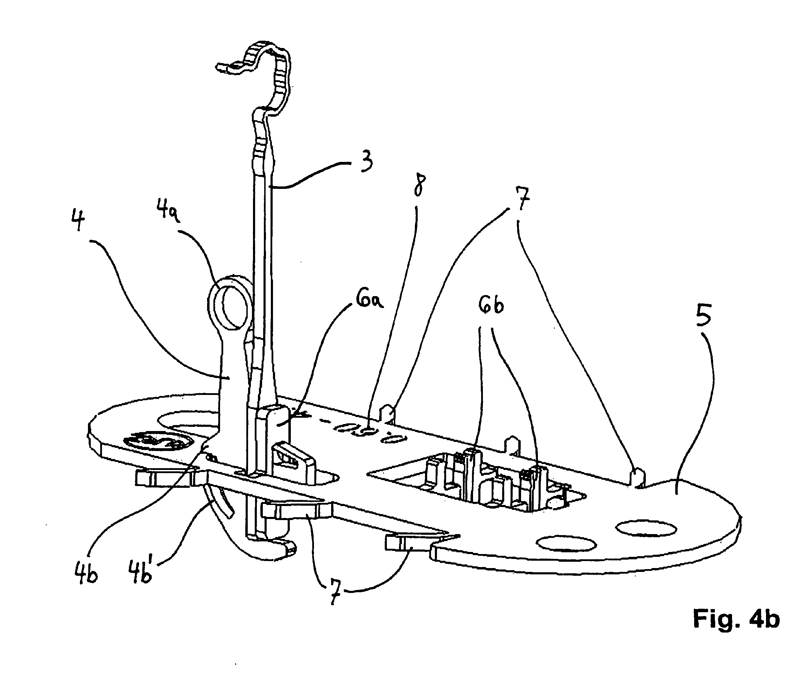

Bei allen in der Zeichnung gezeigten Darstellungen der Prothesenattrappe 3 ist eine **Einrastvorrichtung 4** vorgesehen, mit welcher die Prothesenattrappe 3 in einer - wie in den Figuren 4a und 4b zu erkennen vorzugsweise aufrechten - Bearbeitungsposition fixierbar ist. Mögliche Details der Einrastvorrichtung 4 sind in den Figuren 2a und 2b vergrößert dargestellt, etwa ein laschenförmiges **Bedienelement 4a** oder **Verriegelungselemente 4b, 4b',** die im fixierten Zustand eine Gegenhalterung umgreifen, wie in Fig. 4b gut zu erkennen ist.

Sämtliche in der Zeichnung dargestellten Ausführungsformen der erfindungsgemäßen Testeinrichtung 1 stimmen auch darin überein, dass eine **Halterungsvorrichtung 5** vorgesehen ist, welche die Prothesenattrappe 3 verschwenkbar aufnehmen kann.

Diese Halterungsvorrichtung 5 ist vorzugsweise als Platte aus dünnem Blech gefertigt und von verschiedenen Seiten in der Zeichnung gezeigt.

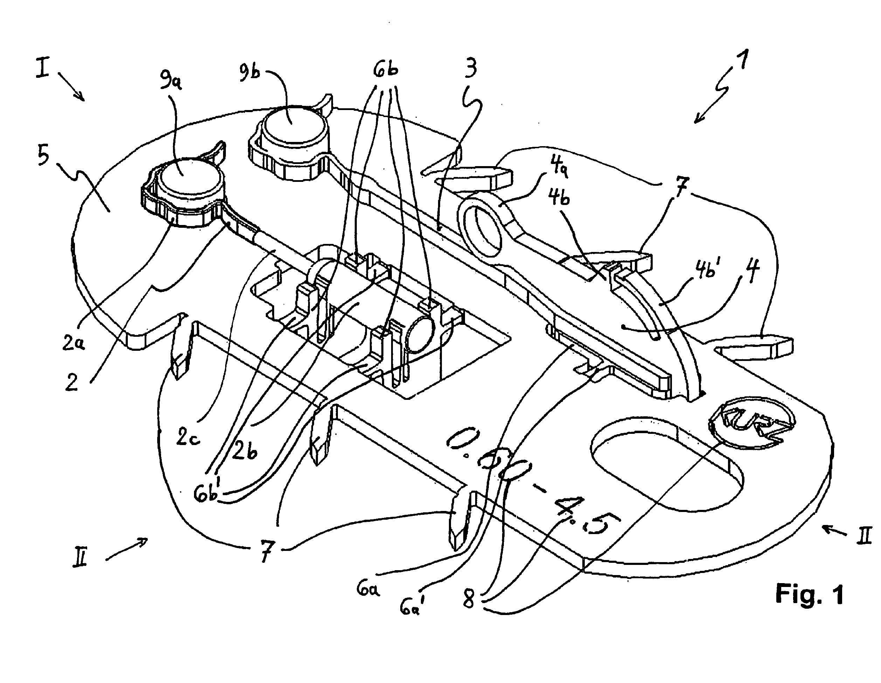

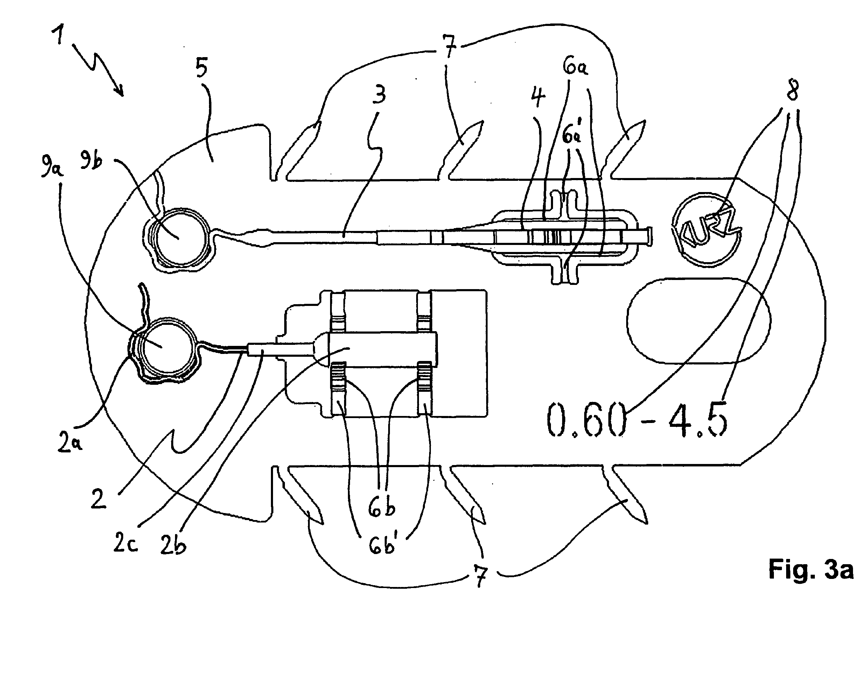

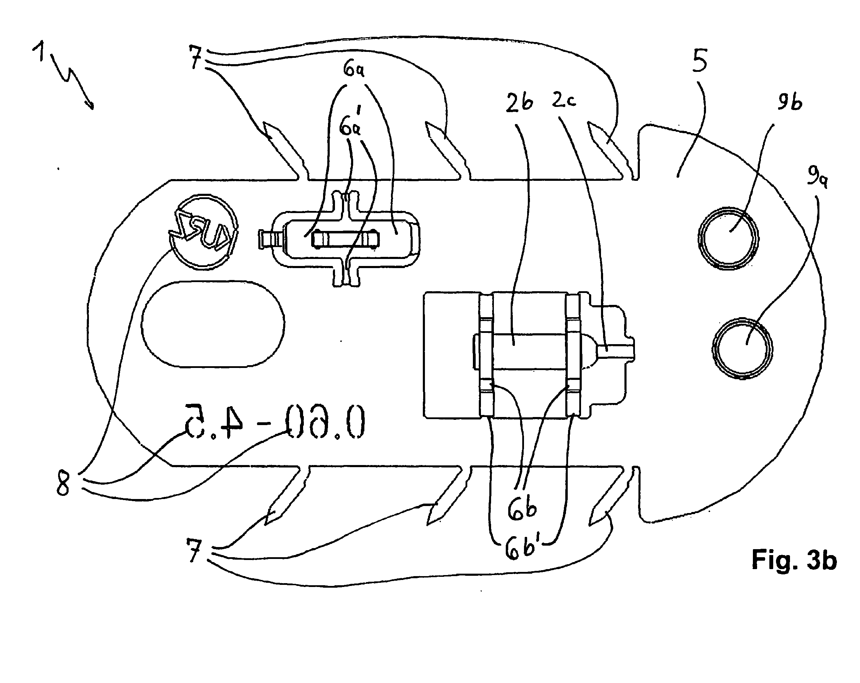

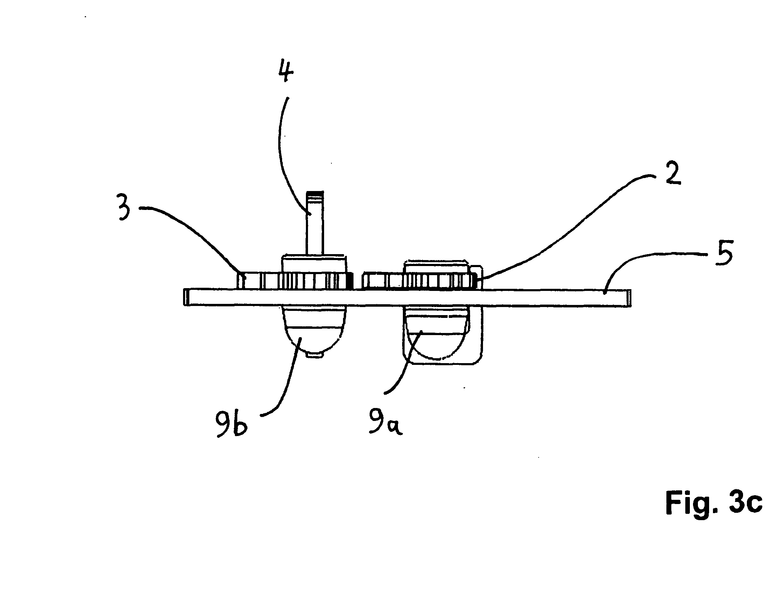

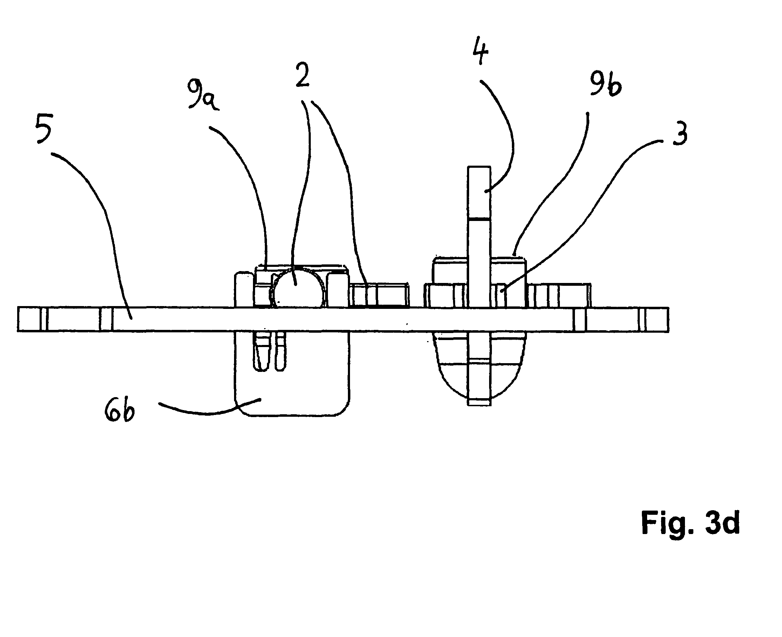

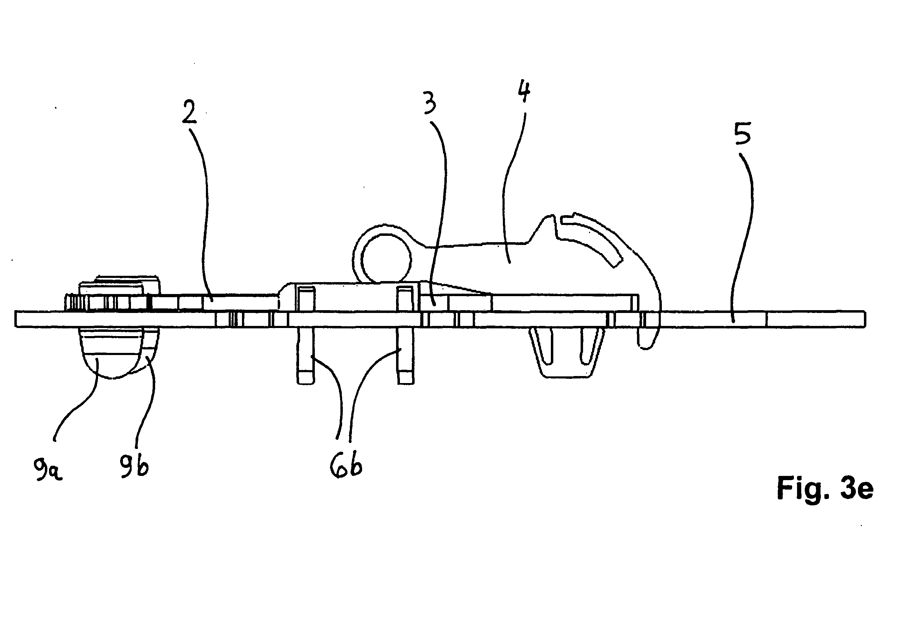

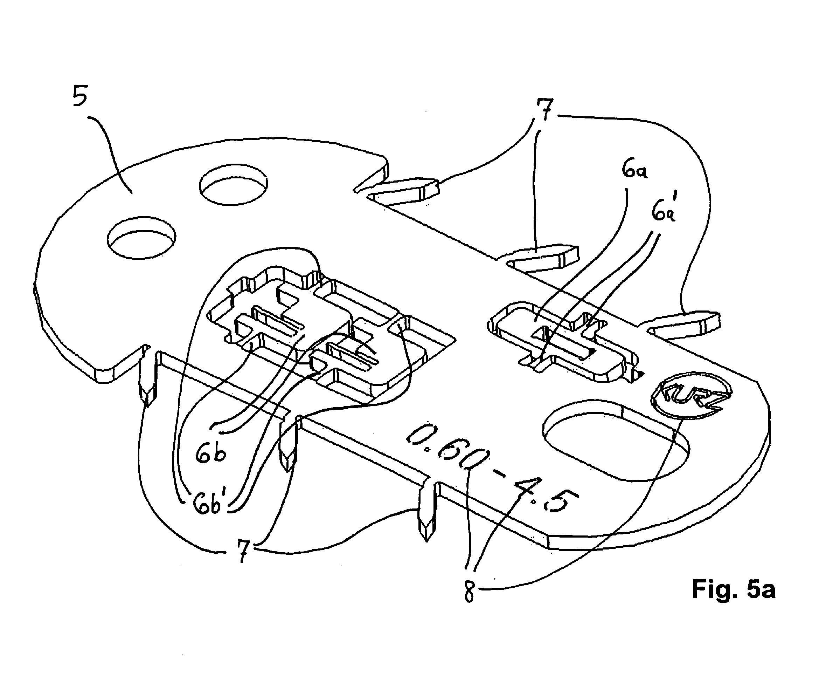

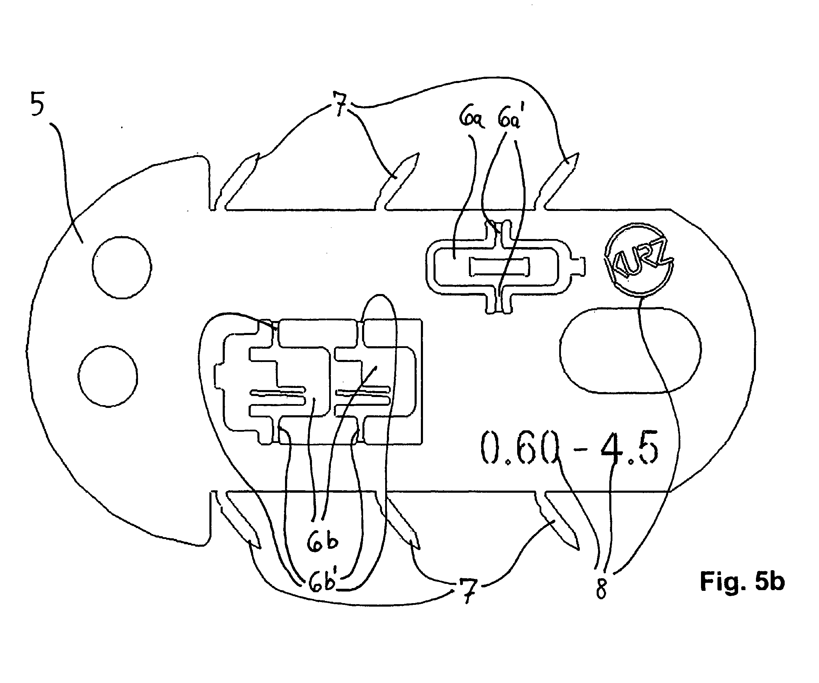

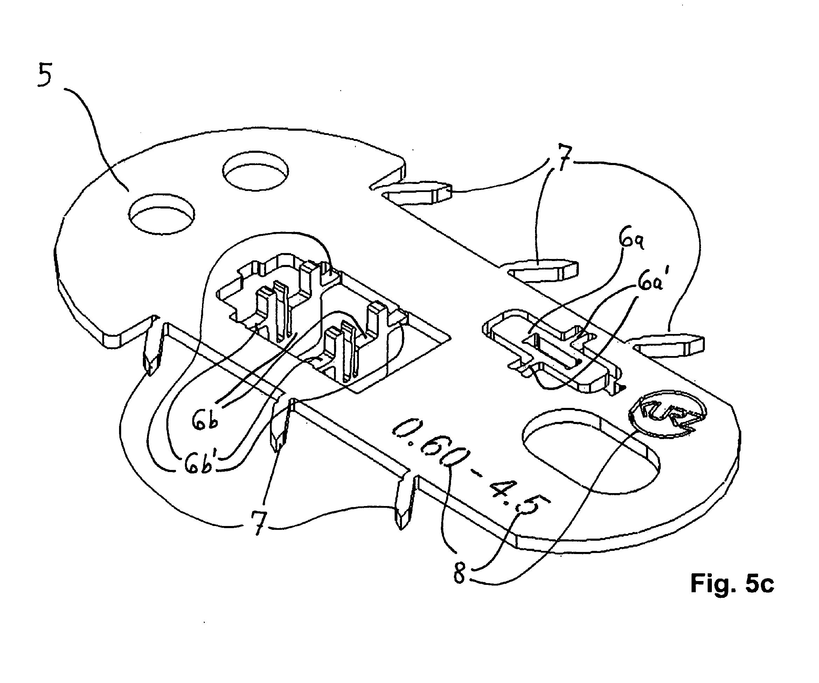

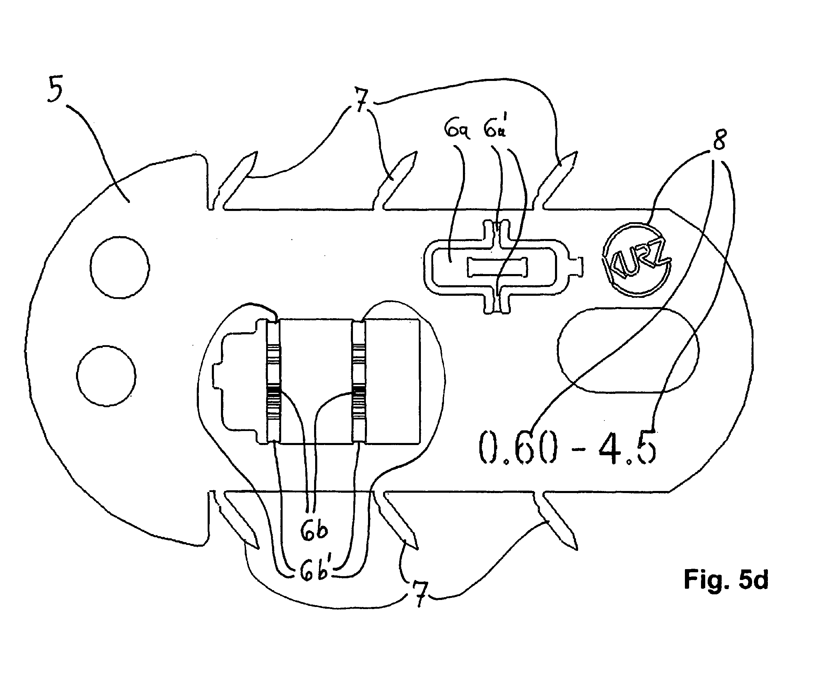

Die Figuren 1 sowie 3a bis 3e zeigen die Platte mit montierter Gehörknöchelchenprothese 2 und Prothesenattrappe 3 im flach liegenden Transportzustand, die Figuren 4a und 4b mit aufgestellter und verrasteter Prothesenattrappe 3 bei bereits entnommener Prothese 2 und die Figuren 5a bis 5d ohne Prothesenattrappe 3 und Prothese 2, wobei die Figuren 5a und 5b speziell den Zustand der Halterungsvorrichtung 5 unmittelbar nach deren Fertigung darstellen, während die Figuren 5c und 5d den Zustand kurz vor der Montage der Gehörknöchelchenprothese 2 auf der Halterungsvorrichtung 5 illustrieren. Nicht dargestellt sind mögliche Ausführungsformen der erfindungs-gemäßen Testeinrichtung, bei welchen die Halterungsvorrichtung derart gestaltet ist, dass sie nur die Prothesenattrappe, nicht aber die zugehörige Gehörknöchelchenprothese aufnehmen kann.

Die Figuren der Zeichnung zeigen eine besonders bevorzugte Ausführungsform der Erfindung, bei der die Halterungsvorrichtung 5 eine mit der Platte über feine **Stege 6a'** verwindbar verbundene **Einhängevorrichtung 6a** zum Einhängen eines Endes der Prothesenattrappe 3 sowie weitere **Einhängevorrichtungen 6b** für die Gehörknöchelchenprothese 2 umfasst. Letztere sind ebenfalls über kleine **Stege 6b'** verwindbar mit der Platte verbunden. Wie oben erwähnt, illustrieren Figuren 5c und 5d insbesondere den Zustand der Halterungsvorrichtung 5 kurz vor der Montage der Gehörknöchelchenprothese 2, wobei die Einhängevorrichtungen 6b für die Aufnahme der Prothese gegenüber dem flach liegenden Zustand in den Figuren 5a und 5b mit Hilfe der Stege 6b' um etwa 90° gegenüber der Platte verdreht sind. Die Prothesenattrappe 3 hingegen wird für den Transport der Testeinrichtung 1 zum Operateur im unverdrehten Zustand der Einhängevorrichtung 6a eingehängt und erst zur Ermittlung der optimalen Bearbeitungsparameter der Gehörknöchelchenprothese 2 gegenüber der Platte um etwa 90° verschwenkt und verrastet, wie in den Figuren 4a und 4b gut zu erkennen ist.

Des Weiteren weist die Halterungsvorrichtung 5 eine Einrichtung zur Transportsicherung auf, welche seitlich von der Halterungsvorrichtung 5 abstehende **grätenförmige Stege 7** umfasst. Diese dienen für die Halterungsvorrichtung 5 mit der aufmontierten, gegen mechanische Beschädigungen relativ empfindlichen Gehörknöchelchenprothese 2 und der ebenfalls aufmontierten Prothesenattrappe 3 als Verrutschsicherung in einer während des Transports zum Operateur verwendeten Umverpackung der Testeinrichtung 1.

Die Halterungsvorrichtung 5 ist bei der gezeigten Ausführungsform der Erfindung optisch kodiert bzw. beschriftet. Die **Kodierung oder Beschriftung 8** enthält technische Informationen bezüglich der Gehörknöchelchenprothese 2 und/oder der zugehörigen Prothesenattrappe 3 sowie Herstellerangaben. Die Kodierung kann auch eine einfache Farbkodierung umfassen. Alternativ oder ergänzend dazu können - bei in der Zeichnung nicht dargestellten Ausführungsformen der Erfindung - auch die Gehörknöchelchenprothese und/oder die zugehörige Prothesenattrappe optisch kodiert und/oder beschriftet sein, wobei die Kodierung oder Beschriftung wiederum technische Informationen über die Gehörknöchelchenprothese und/oder der Prothesenattrappe enthält.

Die technischen Informationen der Kodierung bzw. Beschriftung 8 enthalten unter anderem neben der einer Größenklassifizierung der verwendeten Gehörknöchelchenprothese 2 einen empfohlenen Anfangswert für die elektrische Leistung oder Lichtleistung, welcher zu Beginn der Ermittlung von optimierten Bearbeitungsparametern der intraoperativ thermisch zu behandelnden Teile der Gehörknöchelchenprothese 2 mit Hilfe einer Testbehandlung der Prothesenattrappe 3 angewendet werden soll.

Die Kodierung und/oder Beschriftung 8 wird am einfachsten mittels Laserbehandlung und/oder Anodisierung erzeugt. Ebenso wird die Halterungsvorrichtung 5 selbst bevorzugt durch Laserschneiden der Platte aus einem größeren Blech hergestellt und weiterbearbeitet. Insbesondere die winzigen Strukturen der oben beschriebenen Elemente der Halterungsvorrichtung 5, wie etwa der Einhängevorrichtungen 6a und 6b, der zugehörigen verwindbaren Stege 6a' und 6b' sowie der grätenförmige Stege 7 für die Transportsicherung lassen sich kaum auf andere Weise herstellen.

Zum definierten Offenhalten des ersten Befestigungselements 2a der Gehörknöchelchenprothese 2 sowie des entsprechenden Abschnitts der zugehörigen Prothesenattrappe 3 während des Transports zum Operateur sind bei der gezeigten Ausführungsform der erfindungs-gemäßen Testeinrichtung 1 **Platzhalter-Pins 9a, 9b** in der Halterungsvorrichtung 5 vorgesehen, welche die thermisch vorbehandelten, aus Material mit Formgedächtnis bestehenden Teile in der gewünschten Geometrie fixieren, so dass auch bei einer eventuellen Erwärmung während des Transports ungewünschten Formveränderungen mit Sicherheit verhindert werden können.

Die in den Figuren der Zeichnung dargestellte Ausführungsform der Erfindung zeigt eine Gehörknöchelchenprothese 2, bei welcher das erste Befestigungselement 2a die Form einer Klammer aufweist, die beispielsweise auf den Ambossfortsatz oder auch auf ein anderes Glied der Gehörknöchelchenkette aufgeklipst werden kann. Das zweite Befestigungselement 2b an dem der Klammer entgegen gesetzten Ende ist in diesem Ausführungsbeispiel als Kolben (= Piston) zur direkten Ankopplung der Gehörknöchelchenprothese 2 an das Innenohr ausgebildet.

Bei in der Zeichnung nicht dargestellten Ausführungsformen der Erfindung können die Befestigungselemente 2a, 2b der Gehörknöchelchenprothese 2 geometrisch auch anders gestaltet sein, etwa als Hülse, Schlinge oder Haken. Das erste Befestigungselement 2a kann eine Kopfplatte zur Anlage am Trommelfell sein. Auch kann beispielsweise das zweite Befestigungselement 2b statt als Piston in Form einer Klammer oder stempelförmig zur Auflage auf der Steigbügelfußplatte oder als geschlitzte Glocke zur Befestigung der Gehörknöchelchenprothese 2 auf dem Steigbügel ausgebildet sein.

In weiteren, in der Zeichnung nicht dargestellten Ausführungsformen der Erfindung kann in das Verbindungselement 2c ein Kugelgelenk integriert sein, um eine gewisse postoperative Flexibilität der Gehörknöchelchenprothese 2 zwischen ihren Verbindungsstellen sicherzustellen.

Ebenfalls in der Zeichnung nicht dargestellt sind Ausführungsformen, bei denen die Gehörknöchelchenprothese 2 mit einem aktiven Vibrationsteil eines aktiven, teilweise implantierbaren Hörgeräts verbunden werden kann.

Die Massenverteilung der einzelnen Teile der Gehörknöchelchenprothese 2 kann in Abhängigkeit von einem gewünschten, vorgebbaren Frequenzgang der Schallleitung im Mittelohr derart berechnet sein, dass ein individuelles Tuning der Schallleitungs-Eigenschaften ermöglicht wird.

## Patentansprüche

1. Testeinrichtung (1) mit einer Gehörknöchelchenprothese (2) zur Implantation ins Mittelohr, die mindestens ein Glied oder Teile eines Gliedes der menschlichen Gehörknöchelchenkette ersetzt oder überbrückt, wobei die Gehörknöchelchenprothese (2) an ihrem einen Ende ein erstes Befestigungselement (2a) zur mechanischen Verbindung mit dem Trommelfell oder einem Glied der Gehörknöchelchenkette, insbesondere mit dem Ambossfortsatz oder mit dem Hammergriff, und an ihrem anderen Ende ein zweites Befestigungselement (2b) zur mechanischen Verbindung mit einem weiteren Glied oder Teilen eines Gliedes der Gehörknöchelchenkette oder direkt mit dem Innenohr sowie ein die beiden Befestigungselemente (2a, 2b) Schall leitend miteinander verbindendes Verbindungselement (2c) aufweist, und wobei zumindest Teile der Gehörknöchelchenprothese (2) aus einem Material mit Formgedächtnis (= memory effect) hergestellt sind, welche bei der Implantation der Gehörknöchelchenprothese (2) im Mittelohr einer Form verändernden thermischen Behandlung unterzogen werden,
**dadurch gekennzeichnet,**
**dass** die Testeinrichtung (1) eine Vorrichtung zur Ermittlung einer optimierten Leistungseinstellung und/oder Energieapplikations-Stelle für die Bearbeitung der intraoperativ thermisch zu behandelnden Teile der Gehörknöchelchenprothese (2) umfasst,
und **dass** diese Vorrichtung eine der Gehörknöchelchenprothese (2) nachgebildete Prothesenattrappe (3) enthält, welche zumindest in denjenigen Bereichen, in denen die zugehörige Gehörknöchelchenprothese (2) intraoperativ thermisch behandelt werden soll, mit der Gehörknöchelchenprothese (2) hinsichtlich Material, geometrischer Gestaltung und Herstellungsverfahren identisch aufgebaut ist.

2. Testeinrichtung nach Anspruch 1, **dadurch gekennzeichnet, dass** die Gehörknöchelchenprothese (2) eine Einrichtung zur Variation ihrer Länge aufweist, und dass die Gehörknöchelchenprothese (2) sowie ihre zugehörige Prothesenattrappe (3) zumindest im Bereich der Einrichtung zur Längenvariation aus einem Material mit Formgedächtnis hergestellt sind.

3. Testeinrichtung nach einem der vorhergehenden Ansprüche,
**dadurch gekennzeichnet, dass** die Gehörknöchelchenprothese (2) sowie ihre zugehörige Prothesenattrappe (3) zumindest im Bereich des jeweiligen ersten Befestigungselements (2a) aus einem Material mit Formgedächtnis hergestellt sind.

4. Testeinrichtung nach einem der vorhergehenden Ansprüche,
**dadurch gekennzeichnet, dass** die Gehörknöchelchenprothese (2) sowie ihre zugehörige Prothesenattrappe (3) hinsichtlich Material und geometrischer Gestaltung vollständig identisch aufgebaut sind.

5. Testeinrichtung nach einem der vorhergehenden Ansprüche,
**dadurch gekennzeichnet, dass** die Testeinrichtung (1) eine Einrastvorrichtung (4) enthält, mit welcher die
Prothesenattrappe (3) zumindest in einer Bearbeitungsposition fixierbar ist.

6. Testeinrichtung nach einem der vorhergehenden Ansprüche,
**dadurch gekennzeichnet, dass** eine Halterungsvorrichtung (5) vorgesehen ist, welche die Prothesenattrappe (3) verschwenkbar aufnehmen kann.

7. Testeinrichtung nach Anspruch 6, **dadurch gekennzeichnet, dass** die Halterungsvorrichtung (5) als Platte aus dünnem Blech gefertigt ist und eine mit der Platte verwindbar verbundene Einhängevorrichtung (6a) zum Einhängen eines Endes der Prothesenattrappe (3) umfasst.

8. Testeinrichtung nach einem der Ansprüche 6 oder 7, **dadurch gekennzeichnet, dass** die Halterungsvorrichtung (5) eine Einrichtung zur Transportsicherung aufweist, welche seitlich von der Halterungsvorrichtung (5) abstehende grätenförmige Stege (7) umfasst.

9. Testeinrichtung nach einem der Ansprüche 6 bis 8, **dadurch gekennzeichnet, dass** die Halterungsvorrichtung (5) derart gestaltet ist, dass sie auch die zur Prothesenattrappe (3) gehörende Gehörknöchelchenprothese (2) aufnehmen kann.

10. Testeinrichtung nach einem der Ansprüche 6 bis 9, **dadurch gekennzeichnet, dass** die Halterungsvorrichtung (5) optisch kodiert und/oder beschriftet ist, und dass die Kodierung oder
Beschriftung (8) technische Informationen bezüglich der Gehörknöchelchenprothese (2) und/oder der zugehörigen Prothesenattrappe (3) und/oder Herstellerangaben enthält.

11. Testeinrichtung nach einem der vorhergehenden Ansprüche,
**dadurch gekennzeichnet, dass** die Gehörknöchelchenprothese (2) und/oder die zugehörige Prothesenattrappe (3) optisch kodiert und/oder beschriftet ist, und dass die Kodierung oder Beschriftung technische Informationen über die Gehörknöchelchenprothese (2) und/oder der Prothesenattrappe (3) enthält.

12. Testeinrichtung nach Anspruch 10 oder 11, **dadurch gekennzeichnet, dass** die Kodierung und/oder Beschriftung (8) mittels Laserbehandlung und/oder Anodisierung erzeugt ist.

13. Testeinrichtung nach einem der Ansprüche 10 bis 12, **dadurch gekennzeichnet, dass** die technischen Informationen der Kodierung und/oder Beschriftung (8) einen empfohlenen Anfangswert für die elektrische Leistung oder Lichtleistung enthalten, welcher zu Beginn der Ermittlung von optimierten Bearbeitungsparametern der intraoperativ thermisch zu behandelnden Teile der Gehörknöchelchenprothese (2) mit Hilfe einer Testbehandlung der Prothesenattrappe (3) angewendet werden soll.

14. Testeinrichtung nach einem der vorhergehenden Ansprüche,
**dadurch gekennzeichnet, dass** zumindest Teile der Gehörknöchelchenprothese (2) und der zugehörigen Prothesenattrappe (3) aus einer Nickel-Titan-Legierung, insbesondere aus Nitinol hergestellt sind.

15. Testeinrichtung nach einem der vorhergehenden Ansprüche,
**dadurch gekennzeichnet, dass** die Gehörknöchelchenprothese (2) mit einem aktiven Vibrationsteil eines aktiven, teilweise implantierbaren Hörgeräts verbunden werden kann.

## Claims

1. A test device (1) with an ossicular prosthesis (2) for implantation in the middle ear, which replaces or spans at least one element or parts of an element of the human ossicular chain, the ossicular prosthesis (2) comprising a first fastening element (2a) at one end for mechanical connection to the eardrum or an element of the ossicular chain, specifically to the process of incus or the handle of the malleus, and a second fastening element (2b) at its other end for mechanical connection to another element or parts of an element of the ossicular chain or directly to the inner ear, as well as a connecting element (2c) joining together the two fastening elements (2a, 2b) in such a way as to conduct sound, and at least parts of the ossicular prosthesis (2) being made of a material with shape memory (= memory effect) and being subjected to a shape-modifying heat treatment upon implantation of the ossicular prosthesis (2) in the middle ear, **characterized in that**
the test device (1) includes means for determining an optimized power setting and/or site for the application of energy for processing the parts of the ossicular prosthesis (2) to be intraoperatively heat-treated,
**and in that** these means include a prosthesis dummy (3) which replicates the ossicular prosthesis (2) and which, at least in those areas where the related ossicular prosthesis (2) is to be intraoperatively heat-treated, is identical in structure to the ossicular prosthesis (2) in terms of material, geometric configuration and method of manufacture.

2. A test device according to Claim 1, **characterized in that** the ossicular prosthesis (2) includes means for varying its length, **and in that,** at least in the region of the means for varying the length, the ossicular prosthesis (2) and its related prosthesis dummy (3) are made of a material with memory effect.

3. A test device according to one of the preceding claims, **characterized in that,** at least in the region of their respective first fastening elements (2a), the ossicular prosthesis (2) and its related prosthesis dummy (3) are made of a material with memory effect.

4. A test device according to one of the preceding claims, **characterized in that** the ossicular prosthesis (2) and its related prosthesis dummy (3) are completely identical in structure in terms of material and geometric configuration.

5. A test device according to one of the preceding claims, **characterized in that** the test device (1) includes a snap-in device (4), by means of which the prosthesis dummy (3) can be fixed in at least one position for processing.

6. A test device according to one of the preceding claims, **characterized in that** a holder (5) is provided, which enables the prosthesis dummy (3) to be swivel-mounted.

7. A test device according to Claim 6, **characterized in that** the holder (5) is made in the form of a thin metal plate and includes a mounting device (6a), flexibly connected to the plate, for mounting one end of the prosthesis dummy (3).

8. A test device according to one of Claims 6 or 7, **characterized in that** the holder (5) has means comprising rib-like elements (7) projecting from the sides of the holder (5) for its safety during transportation.

9. A test device according to one of Claims 6 to 8, **characterized in that** the holder (5) is designed in such a way that it can also accommodate the ossicular prosthesis (2) belonging with the prosthesis dummy (3).

10. A test device according to one of Claims 6 to 9, **characterized in that** the holder (5) is optically coded and/or inscribed, **and in that** the coding or inscription (8) includes technical data regarding the ossicular prosthesis (2) and/or the related prosthesis dummy (3) and/or details relating to the manufacturer.

11. A test device according to one of the preceding claims, **characterized in that** the ossicular prosthesis (2) and/or related prosthesis dummy (3) is optically coded and/or inscribed, **and in that** the coding or inscription includes technical data regarding the ossicular prosthesis (2) and/or the prosthesis dummy (3).

12. A test device according to Claim 10 or 11, **characterized in that** the coding and/or inscription (8) is produced by a laser and/or anodizing process.

13. A test device according to one of Claims 10 to 12, **characterized in that** the technical data of the coding and/or inscription (8) include a recommended initial value for the electrical power or light power, this value to be applied, with the aid of a test treatment of the prosthesis dummy (3), at the start of the procedure for determining optimized processing parameters in respect of the parts of the ossicular prosthesis (2) to be intraoperatively heat-treated.

14. A test device according to one of the preceding claims, **characterized in that** at least parts of the ossicular prosthesis (2) and related prosthesis dummy (3) are made of a nickel/titanium alloy, specifically nitinol.

15. A test device according to one of the preceding claims, **characterized in that** the ossicular prosthesis (2) can be connected to an active vibrating component of an active, partially implantable hearing aid.

## Revendications

1. Dispositif d'essai (1) avec une prothèse d'osselets d'oreille (2) pour implantation dans l'oreille moyenne, qui remplace ou joint un maillon ou des parties d'un maillon de la chaîne d'osselets humaine de l'oreille, dans lequel la prothèse d'osselets d'oreille (2) présente, à sa première extrémité, un premier élément de fixation (2a) pour la liaison mécanique avec le tympan ou un maillon de la chaîne d'osselets de l'oreille, en particulier avec le prolongement de l'enclume ou avec la prise du marteau, et, à son autre extrémité, un second élément de fixation pour la liaison mécanique avec un autre maillon ou des parties d'un maillon de la chaîne d'osselets de l'oreille ou directement avec l'oreille interne ainsi qu'un élément de liaison (2c) liant l'un à l'autre les deux éléments de fixation (2a, 2b) de façon à conduire le son, et dans lequel au moins des parties de la prothèse d'osselets d'oreille (2) sont fabriquées dans un matériau à mémoire de forme (ou memory effect), lesquelles parties sont soumises à un traitement thermique modificateur de forme lors de l'implantation de la prothèse d'osselet d'oreille (2) dans l'oreille moyenne,
**caractérisé en ce que**
le dispositif d'essai (1) comprend un dispositif pour
établir une position de performance optimisée et/ou une position d'application d'énergie optimisée pour le travail des parties de la prothèse d'osselets d'oreille (2) à traiter thermiquement intra-opération et **en ce que** ce dispositif contient une simili-prothèse (3) conformée comme la prothèse d'osselets d'oreille (2), laquelle simili-prothèse est conçue au moins dans les zones, dans lesquelles la prothèse d'osselets d'oreille (2) afférente doit être traitée thermiquement intra-opération, de manière identique à la prothèse d'osselets d'oreille (2) en matière de matériau, de conformation géométrique et de procédé de fabrication.

2. Dispositif d'essai selon la revendication 1, **caractérisé en ce que** la prothèse d'osselets d'oreille (2) présente un dispositif pour faire varier sa longueur et **en ce que** la prothèse d'osselets d'oreille (2) ainsi que sa simili-prothèse (3) afférente sont fabriquées dans un matériau à mémoire de forme au moins dans la zone du dispositif où l'on fait varier sa longueur.

3. Dispositif d'essai selon l'une quelconque des revendications précédentes, **caractérisé en ce que** la prothèse d'osselets d'oreille (2) ainsi que sa simili-prothèse (3) afférente sont fabriquées dans un matériau à mémoire de forme au moins dans la zone du premier élément de fixation respectif (2a).

4. Dispositif d'essai selon l'une quelconque des revendications précédentes, **caractérisé en ce que** la prothèse d'osselets d'oreille (2) ainsi que sa simili-prothèse (3) afférente sont fabriquées de manière tout à fait identique en matière de matériau et de conformation géométrique.

5. Dispositif d'essai selon l'une quelconque des revendications précédentes, **caractérisé en ce que** le dispositif d'essai contient un dispositif d'insertion (4) avec lequel la simili-prothèse (3) peut être fixée au moins dans une position de travail.

6. Dispositif d'essai selon l'une quelconque des revendications précédentes, **caractérisé en ce qu'**il est prévu un dispositif de support (5) qui peut recevoir la simili-prothèse (3) à pivotement.

7. Dispositif d'essai selon la revendication 6, **caractérisé en ce que** le dispositif de support (5) est fabriqué sous forme de plaque en tôle mince et comprend un dispositif de suspension (6a) relié à la plaque de manière à pouvoir se tordre avec celle-ci pour suspendre une extrémité de la simili-prothèse (3).

8. Dispositif d'essai selon l'une quelconque des revendications 6 ou 7, **caractérisé en ce que** le dispositif de support (5) présente un dispositif pour sécuriser le transport, qui comprend des barrettes (7) en forme de grille faisant saillie latéralement du dispositif de support (5).

9. Dispositif d'essai selon l'une quelconque des revendications 6 à 8, **caractérisé en ce que** le dispositif de support (5) est conformé de sorte qu'il puisse recevoir également la prothèse d'osselets d'oreille (2) appartenant à la simili-prothèse (3).

10. Dispositif d'essai selon l'une quelconque des revendications 6 à 9, **caractérisé en ce que** le dispositif de support (5) est codé optiquement et/ou marqué et **en ce que** le codage ou la marque (8) contient des informations techniques en matière de prothèse d'osselets d'oreille (2) et/ou de simili-prothèse (3) afférente et/ou des indications du fabricant.

11. Dispositif d'essai selon l'une quelconque des revendications précédentes, **caractérisé en ce que** la prothèse d'osselets d'oreille (2) et/ou la simili-prothèse (3) afférente et/ou sont codés optiquement et/ou marqués et **en ce que** le codage ou le marquage contient des informations techniques sur la prothèse d'osselets d'oreille (2) et/ou la simili-prothèse (3).

12. Dispositif d'essai selon la revendication 10 ou la revendication 11, **caractérisé en ce que** le codage et/ou le marquage (8) est ou sont produits par traitement au laser et/ou anodisation.

13. Dispositif d'essai selon l'une quelconque des revendications 10 à 12, **caractérisé en ce que** les informations techniques du codage et/ou du marquage (8) contiennent une valeur initiale recommandée pour la puissance électrique ou le flux lumineux, qui doit être appliquée au départ à l'établissement de paramètres de travail optimisés des parties de la prothèse d'osselets d'oreille (2) à traiter par voie thermique intra-opération à l'aide d'un traitement d'essai de la simili-prothèse (3).

14. Dispositif d'essai selon l'une quelconque des revendications précédentes, **caractérisé en ce qu'**au moins des parties de la prothèse d'osselets d'oreille (2) et de la simili-prothèse (3) afférente sont fabriquées en alliage de nickel-titane, en particulier en Nitinol.

15. Dispositif d'essai selon l'une quelconque des revendications précédentes, **caractérisé en ce que** la prothèse d'osselets d'oreille (2) peut être liée à une pièce vibrante active d'un appareil actif de correction auditive en partie implanté.
